(19) Europäisches Patentamt
European Patent Office
Office européen des brevets

(11) **EP 4 506 709 A2**

(12) **EUROPEAN PATENT APPLICATION**

(43) Date of publication:
**12.02.2025 Bulletin 2025/07**

(51) International Patent Classification (IPC):
***G01R 33/58*** (2006.01)   ***A61N 5/10*** (2006.01)

(21) Application number: **24189582.0**

(52) Cooperative Patent Classification (CPC):
**G01R 33/58; A61N 5/1049;** A61N 2005/1055

(22) Date of filing: **18.07.2024**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.07.2023 GB 202311217**

(71) Applicant: **Elekta Limited
Crawley, Sussex
RH10 9RR (GB)**

(72) Inventor: **FREEDMAN, Joshua
Crawley (GB)**

(74) Representative: **Beal, James Michael et al
Kilburn & Strode LLP
Lacon London
84 Theobalds Road
London WC1X 8NL (GB)**

(54) **IMAGE DISTORTION IN RADIOTHERAPY**

(57) A method for evaluating contour display accuracy for radiotherapy, a computer-readable medium and a phantom for use in evaluating contour display accuracy for radiotherapy are provided. The method includes obtaining, by a control device, a through-plane distorted MR image comprising a representation of a phantom disposed on a patient support surface of a radiotherapy device. The method further includes determining, by the control device, a difference between the representation of the phantom and a reference contour of the phantom.

Fig. 10a

EP 4 506 709 A2

## Description

This disclosure relates to radiotherapy, and in particular to evaluating contour display accuracy and distortion of images for radiotherapy.

## Background

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises a gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

In radiotherapy treatment, it is desirable to deliver a prescribed dose of radiation to a target region of a subject and to limit irradiation of other parts of the subject, i.e. of healthy tissue. Motion of the subject can cause a decreased dose to be applied to the target region and/or an increased dose to be applied to the healthy tissue. To address this, known techniques include monitoring a location of the subject and gating the treatment beam such that radiation is applied only when the subject (i.e. the target region within the subject) is in a desired location and not when the subject/target region is in a suboptimal location. Alternatively, or in addition, tracking can be used to move the beam such that it continues to coincide with the target region as the target region moves. Doing so ensures that the dose actually delivered to the target and surrounding healthy tissue best corresponds to the intended treatment plan.

Imaging of a subject before or during a radiotherapy treatment can provide up-to-date anatomical location information, for example regarding a tumour or organs at risk (OAR). Magnetic resonance (MR) imaging is one imaging modality that can be used for this purpose. A radiotherapy device may comprise or be coupled to an MR imaging apparatus, which enables imaging of a subject during treatment and/or in a treatment position before treatment commences. In other words, the radiotherapy device may be an MR-linac. MR imaging enables detailed visualisation of the internal anatomy of a subject with good soft tissue contrast. MR imaging involves placing the subject in a strong magnetic field. Localisation of measurement signals to different parts of the anatomy of the subject can be achieved by varying the magnetic field present at different spatial locations, i.e. by introducing one or more magnetic field gradients. However, it can be difficult to achieve a perfectly linear magnetic field gradient. Non-linearities in the magnetic field gradient lead to distortions in the localisation of measurement signals to different parts of the subject anatomy. In other words, the image that is obtained may be geometrically distorted relative to the underlying subject anatomy. Due to this, there may be geometrical differences between reference images of subject anatomy, e.g. those that are used in treatment planning, and the distorted images that are later obtained.

There is a need for improved evaluation of image distortion and improved evaluation of corrections that are applied to compensate for this. In addition, there is a need for improved testing of correspondence between structures from different images. Accordingly, there is a need for improved determination of the accuracy of imaging and the accuracy of image processing in radiotherapy.

The present invention seeks to address these and other disadvantages encountered in the prior art.

## Summary

An invention is set out in the independent claims.

According to an aspect, there is provided a method for evaluating contour display accuracy for radiotherapy. The method comprises obtaining, by a control device, a through-plane distorted MR image comprising a representation of a phantom disposed on a patient support surface of a radiotherapy device. The method further comprises determining, by the control device, a difference between the representation of the phantom and a reference contour of the phantom.

According to a further aspect, there is provided a computer-readable medium comprising computer-executable instructions which, when executed by a processor, cause performance of the above-mentioned method.

According to a further aspect, there is provided a phantom for use in evaluating contour display accuracy for radiotherapy. The phantom comprises an outer surface tapered along at least one axis of the phantom between a base and a tapered end. The phantom further comprises a fixation mechanism configured to fix the phantom on a patient support surface of a radiotherapy device such that the tapered end is selectively alignable at different respective times with each of three perpendicular axes of the radiotherapy device.

## Brief Description of the Drawings

Specific embodiments are now described, by way of example only, with reference to the drawings, in which:

Figure 1 depicts a radiotherapy device or apparatus according to the present disclosure;
Figure 2 depicts a schematic of an MR imaging apparatus according to the present disclosure;

Figure 3 depicts a magnetic field gradient according to the present disclosure;

Figure 4a depicts an arrangement of phantoms according to the present disclosure;

Figure 4b depicts an image comparison according to the present disclosure;

Figure 5 depicts a phantom according to the present disclosure;

Figure 6 depicts the phantom of Figure 5 in a reoriented position according to the present disclosure;

Figure 7 depicts an alternative phantom according to the present disclosure;

Figure 8 depicts a further alternative phantom according to the present disclosure;

Figure 9 depicts a method according to the present disclosure;

Figure 10a depicts an image comparison according to the present disclosure;

Figure 10b depicts a further image comparison according to the present disclosure;

Figure 11 depicts a block diagram of one implementation of a radiotherapy system according to the present disclosure;

Figure 12 depicts a computer program product according to the present disclosure.

Detailed Description

The current disclosure provides a method for evaluating contour display accuracy for radiotherapy. The method includes obtaining, by a control device, a through-plane distorted MR image comprising a representation of a phantom disposed on a patient support surface of a radiotherapy device. The method further includes determining, by the control device, a difference between the representation of the phantom and a reference contour of the phantom. The reference contour can be, for example, a ground-truth contour of the phantom without distortion or a distortion-corrected contour of the phantom. The method enables the amount of through-plane distortion and its effect on representations of particular imaged structures to be determined. It also enables the distortion/correction/warping of contours, which may be performed to compensate for the known or expected distortion, to be evaluated. This enables a robust determination of whether images and distortion in radiotherapy are being processed in a consistent and accurate way. By performing this method using a contour and representation of a phantom, the accuracy of the imaging and the image processing can be tested so as to improve patient safety.

The current disclosure also provides a phantom for evaluating contour display accuracy for radiotherapy. The phantom includes an outer surface tapered along at least one axis of the phantom between a base and a tapered end. The phantom further includes a fixation mechanism which is configured to fix the phantom on a patient support surface of a radiotherapy device such that the tapered end is selectively alignable at different respective times with each of three perpendicular axes of the radiotherapy device. The phantom can be used in the above-mentioned method. Because the outer surface of the phantom tapers or narrows along an axis of the phantom, an imaged plane taken perpendicular to this axis which exhibits through-plane distortion will lead to the representation of the phantom in the image being distorted relative to its known shape. This therefore enables through-plane distortion and image processing performed to compensate for this to be evaluated. Moreover, because the phantom includes a fixation mechanism for fixing the phantom such that the tapered end is aligned with different axes at different times, using this phantom the through-plane distortion and compensatory image processing can be evaluated reliably and reproducibly for images taken in multiple different planes because the direction of taper can be aligned with the different axes for the acquisition of the differently oriented imaging planes.

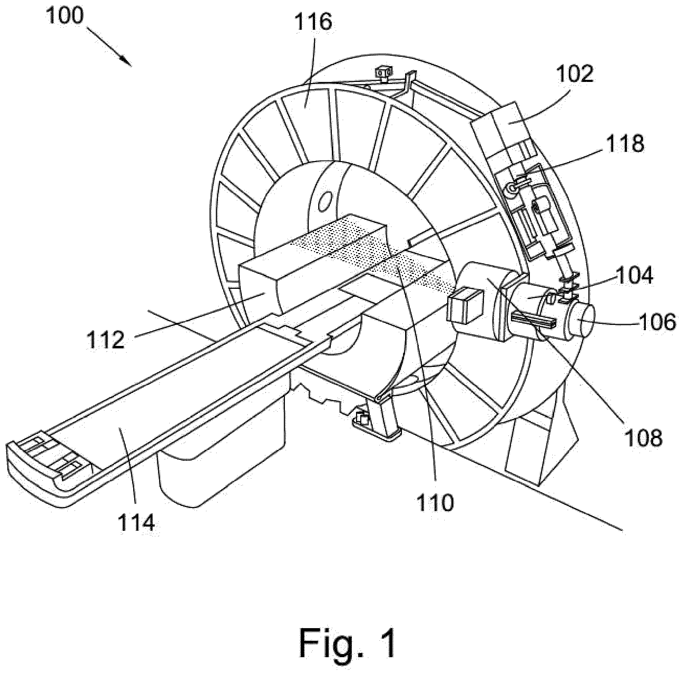

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in Figure 1 is in accordance with the present disclosure and is suitable for use with the disclosed systems and apparatuses. While the device in Figure 1 is an MR-linac, implementations of the present disclosure may be any radiotherapy device, for example a linac device.

The device 100 depicted in Figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in Figure 1 comprises a source of radiofrequency waves 102, a waveguide 104, a source of electrons 106, a collimator 108 such as a multi-leaf collimator configured to collimate and shape the beam 110, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a control device or processor. The control device is also communicatively coupled to a memory device comprising computer-executable instructions which may be executed by the control device. As used herein, a control device may also be referred to as a controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move to different positions including a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by one or more support surface actuators, which may be described as an actuation mechanism. The actuation mechanism is configured to move the support surface at least in a direction parallel to, and defined by, the central axis of the bore. The terms subject and patient are used interchangeably herein such that the patient support surface can also be described as a subject support surface. The support surface may also be referred to as a moveable or adjustable couch or table or as a patient couch or subject couch.

The radiotherapy apparatus/device depicted in Figure 1 also comprises MR imaging apparatus 112. The MR imaging apparatus 112 is configured to obtain images of a subject positioned, i.e. located, on the patient support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the control device. While the discussion herein may focus on MR imaging by way of example, alternatively or in addition to MR imaging, one or more other imaging techniques, modalities, sensors or detectors may be used, such as CT/X-ray, PET, optical imaging/cameras, infra-red imaging, ultrasound imaging or time-of-flight techniques. Any one or more of these may be used to generate images of the subject and/or to determine the position of the target.

The control device is a computer, processor, or other processing apparatus. The control device may be formed by several discrete processors; for example, the control device may comprise a processor for each of the various individual components of the radiotherapy device as described herein. The control device is communicatively coupled to a memory, e.g. a computer readable medium. The control device may be communicatively coupled to one, multiple or all of the various individual components of the radiotherapy device as described herein.

The linac device also comprises several other components and systems as will be understood by the skilled person. For example, in order to ensure the linac does not leak radiation, appropriate shielding is also provided.

The radiotherapy device and/or the control device may be configured to perform any of the method steps presently disclosed and may comprise computer executable instructions which, when executed by a processor cause the processor to perform any of the method steps presently disclosed, or when executed by the control device cause the control device to perform any of the method steps presently disclosed, or when executed by the radiotherapy device cause the radiotherapy device to perform any of the method steps presently disclosed. Any of the steps that the radiotherapy device and/or the control device is configured to perform may be considered as method steps of the present disclosure and may be embodied in computer executable instructions for execution by a processor. A computer-readable medium may comprise the above-described computer executable instructions.

Referring to Figure 1, axes may be defined as follows. The z-axis may be defined as pointing into/out of the bore of the gantry 116 and concentric with the gantry 116. The x-axis may be defined as pointing horizontally left/right relative to the centre of the bore. The y-axis may be defined as pointing vertically up/down relative to the centre of the bore.

Anatomical positions within a subject are typically defined by reference to coronal, sagittal and transverse planes. The coronal plane divides the body into front and back portions. The sagittal plane divides the body into left and right portions. The transverse plane divides the body into head and tail portions. For a subject lying on the patient support surface 114 of Figure 1, the coronal plane is defined in the x-z plane of the radiotherapy device. Similarly, the sagittal plane is defined in the y-z plane of the radiotherapy device and the transverse plane is defined in the x-y plane of the radiotherapy device.

A treatment plan for a subject may comprise information specifying the radiotherapy machine parameters to be used for treating the subject. This is based on the subject being disposed in a particular position within or relative to the radiotherapy device. For example, this may be based on one or more pretreatment or template images of the subject. These machine parameters may be optimised for delivering a particular radiative dose to the anatomical position of the target (tumour) within the subject and avoiding delivering substantial dose to healthy tissue within the subject. However, during a radiotherapy treatment session, the subject or parts of the subject may move, for example due to breathing, cardiac motion, coughing, twitching, etc. Images of the subject may be generated during the treatment session in order to provide a real-time, or close to real-time, check on the position of the subject or parts of the subject. This can be used to determine whether/when the subject is in a suitable position for delivering radiation to the target.

MR imaging is an imaging technique which involves placing a subject in a strong magnetic field which aligns the magnetic moments of protons in the subject to produce a net magnetization. Irradiating the subject with radiofrequency (RF) pulses of a particular resonant frequency tips the net magnetization of these protons by a flip-angle $\alpha°$ into a plane perpendicular to the strong magnetic field. Immediately after the RF pulse is completed, the tipped net magnetization of these protons realigns with the strong magnetic field. The changing magnetic flux generated during realignment induces a voltage in a coil. This is measured and analysed to provide information on the distribution of different tissues within the subject. While the skilled person will be familiar with MR imaging techniques, the following explanation of the general principles is provided below.

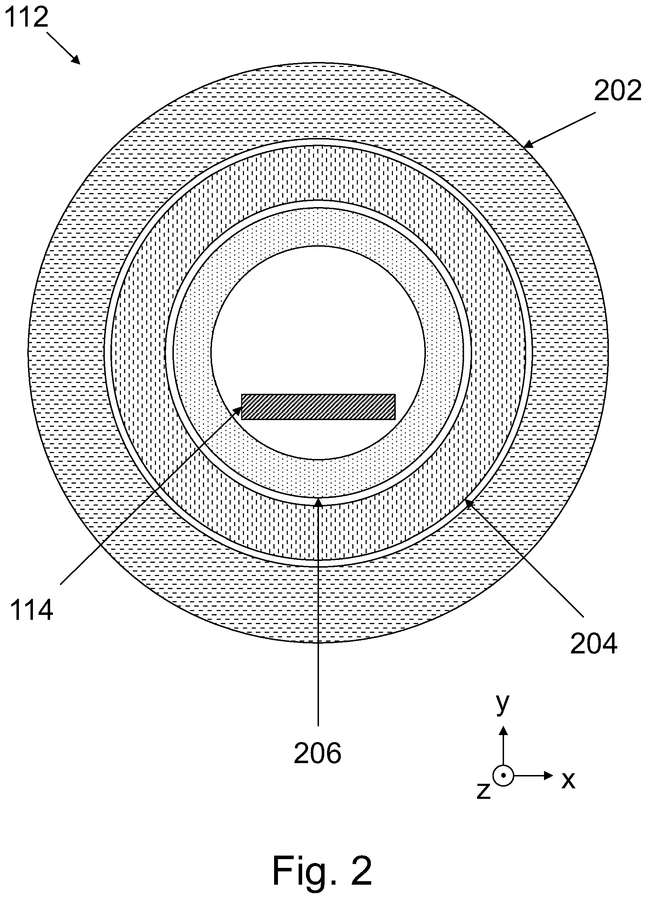

Figure 2 depicts a schematic of an MR imaging apparatus 112. The MR imaging apparatus 112 may be comprised in the device 100. Figure 2 depicts a view of the MR imaging apparatus 112 taken along the bore of the MR imaging device 112 and/or of the gantry 116. This bore is depicted as being oriented along the z-axis. The end of the patient support surface 114 is also depicted in Figure 2. As shown in Figure 2, the MR imaging apparatus 112 comprises a primary magnetic field coil 202, a magnetic gradient coil 204 and a radiofrequency (RF) coil 206. Each of these may have cylindrical symmetry around the bore and each may be positioned concentrically around the bore.

The primary magnetic field coil 202 is configured to produce a constant magnetic field along the bore of the MR imaging apparatus. This constant magnetic field is oriented in the z-direction in Figure 2. This constant magnetic field coil is of high strength, typically of the order of one or more Tesla.

The magnetic gradient coil 204 is configured to generate a spatially varying magnetic field in the volume of the bore, i.e. within a subject positioned on the patient support surface 114. The spatially varying magnetic field is in the same direction as the constant magnetic field produced by the primary magnetic field coil 202, i.e. in the z-direction. However, this magnetic field in the z-direction may vary (in strength) in dependence on location along x, y or z axes. The magnetic gradient coil 204 may include an x-direction magnetic gradient coil and/or a y-direction magnetic gradient coil and/or a z-direction magnetic gradient coil. Each of these gradient coils may be configured to generate a spatially varying field in the z-direction which varies along a particular axis. For example, the x-direction magnetic gradient coil may generate a magnetic field in the z-direction which varies along the x-axis. Similarly, the y-direction magnetic gradient coil may generate a magnetic field in the z-direction which varies along the y-axis. The z-direction magnetic gradient coil may generate a magnetic field in the z-direction which varies along the z-axis.

The RF coil 206 is configured to generate RF pulses and to detect MR signals produced in the subject in response to these RF pulses. When a current is passed through the RF coil 206, an oscillating/rotating magnetic field is produced in the bore. This magnetic field is perpendicular to the constant magnetic field produced by the primary magnetic field coil 202. Conversely, changing magnetic fields of the protons of the subject induce a voltage in the RF coil 206 which can be analysed to derive information on the distribution of tissue within the subject.

The RF coil 206 may comprise a transmitter coil configured to generate the RF pulses and a receiver coil configured to detect the MR signals. Alternatively, the same coil may be used for both transmitting and receiving.

The MR imaging apparatus 112 may additionally comprise one or more of thermal insulation, magnetic shielding and RF shielding. These may be disposed between and/or around the outside of one or more of the coils described above. The MR imaging apparatus 112 may comprise an MR controller which controls the MR imaging apparatus 112. The MR controller may be a computer, processor, or other processing apparatus. The MR controller may be communicatively coupled to a memory, e.g. a computer readable medium. The MR controller may be communicatively coupled to one or more other components of the device 100 and/or to one or more controllers thereof.

In operation, MR images are generated using a pulse sequence, which may be stored in or communicated to the MR controller. The pulse sequence includes gradient pulses to be applied by the magnetic gradient coil 204. The pulse sequence also includes RF pulses to be applied by the RF coil 206. Furthermore, the pulse sequence may include signal acquisition operations, which may be applied using an analog-to-digital converter. The pulse sequence may define properties of the gradient pulses and/or of the RF pulses. These properties may include one or more of amplitude, timing, frequency, phase and duration.

In MR imaging, the response of hydrogen nuclei in water molecules within the subject to the pulse sequence can be used to generate images of the subject. A hydrogen nucleus, i.e. a proton, may be described as a rotating positive charge, the rotation of which generates a magnetic field. This magnetic field is referred to as a magnetic moment. The magnetic moments can only occupy one of two quantum states, known as spin-up or spin-down. When no magnetic field is applied, magnetic moments are randomly orientated due to non-uniform thermodynamic fluctuations. The constant magnetic field generated by the primary magnetic field coil 202 acts to align and anti-align the magnetic moments in the subject with this constant magnetic field. Overall, a net magnetization can be generated as there is typically an excess of magnetic moments aligned (i.e. spin-up) than anti-aligned (i.e. spin-down) with the magnetic field. Alignment and anti-alignment is possible due to the interaction of the constant magnetic field with each proton, which creates a torque that causes it to precess around the direction of the constant magnetic field, i.e. the z-direction. The frequency $\omega_0$ of this precession is equal to the constant magnetic field strength $B_0$ multiplied by a constant $\gamma$ known as the gyromagnetic ratio. This gyromagnetic ratio has different known values depending on the nucleus being considered. For Hydrogen nuclei, the gyromagnetic ratio is $2.67 \times 10^8$ rad s$^{-1}$ T$^{-1}$. The Larmor frequency $\omega_0$ of precession of Hydrogen nuclei in the subject can therefore be calculated for a particular ap-

plied constant magnetic field $B_0$. Applying an RF pulse at the Larmor frequency tips the net magnetization of the hydrogen nuclei through a flip-angle $\alpha°$ (into the transverse plane). Immediately after the RF pulse is completed, the tipped net magnetization realigns with the constant magnetic field. This produces an MR signal at the Larmor frequency, which induces a measurable voltage in the RF coil 206.

The above mechanism provides means for detecting the quantity of hydrogen nuclei (protons) in the sample, and therefore for determining information about the composition of the sample. However, it does not discriminate between protons in one part of a subject and protons in another part of the subject. In other words, it provides means for determining a measure of the total number of hydrogen nuclei in the subject, but not for providing an image mapping these spatially. Techniques enabling signals arising from different locations to be discriminated may be referred to as spatial encoding. Examples of such techniques will be described below.

In order to discriminate between nuclei located at different locations, the magnetic gradient coil 204 is used to vary the magnetic field spatially. For example, the z-direction magnetic gradient coil may generate a magnetic field that increases from a negative value at a first end of the bore to zero in the centre of the bore to a positive value at a second, opposite, end of the bore. The total field experienced by the nuclei is then the sum of the constant magnetic field $B_0$ and this spatially varying magnetic field. An RF pulse applied by the RF coil 206 may have a certain narrow bandwidth around the Larmor frequency. The nuclei at the centre of the bore will experience a field causing them to precess at this same Larmor frequency, since the spatially varying field is zero at that point, and resonance will therefore occur causing a measurable MR signal to be produced. Towards the first or second end of the bore, the magnetic field, and therefore the frequency of precession, will be smaller or larger respectively, meaning that resonance with the applied RF pulse will not occur and a measurable signal will not be produced from these regions. In effect, a central region of the bore (in the z-direction) has been interrogated by the applied signals. Therefore, applying a magnetic field gradient in a given direction provides means for selecting a particular spatial slice along that direction for measurement. For the magnetic field varying in the z-direction as described above, this spatial slice will be in the x-y plane and will have a slice thickness in the z-direction. Varying the carrier frequency of the applied RF pulse in the presence of the spatially varying magnetic field enables different slices along this direction to be measured. Applying gradients in a different direction would enable spatially selective measurements to be taken along those respective directions.

While application of a gradient enables a particular slice of the subject to be selected for measurement, this alone does not provide measurements discriminating between different locations in the selected slice. Such 'in-plane' measurements can be performed using phase-encoding and frequency encoding. In the above example, these in-plane measurements are in the x-y plane.

Phase encoding can be applied in a direction perpendicular to the applied gradient, e.g. in the x-direction. A phase-encode gradient can be applied by varying the magnetic field along the x-direction. This causes the precession of the nuclei to increase or decrease in frequency depending on their location along the x-axis (i.e. from the left to the right of the bore as shown in Figure 2). The spins of the nuclei are thereby caused to dephase to different extents. When the phase-encode gradient is turned off, the protons return to their original precession frequency, but retain this dephasing. However, protons at certain intervals along the x-direction will remain in phase due to the dephasing at these intervals being 360°, meaning that the protons at these intervals effectively remain in phase. The signals from these protons will add together rather than cancelling out and will therefore be measurable. Thus, applying a particular phase-encode gradient along the x-direction can be used to measure a sample at a particular interval or spatial frequency along the x-direction. The protons at that interval are picked out for measurement by the filter or comb that the application of the phase-encode gradient results in. Applying the RF signal multiple times, each time followed by application of a different respective phase-encode gradient, leads to a different filter or comb being applied since protons at different intervals will be picked out through being 360° out of phase (i.e. in phase). This enables different spatial locations in the x-direction to be measured.

To obtain measurements in the second in-plane direction, i.e. in the y-direction, frequency encoding can be used. A gradient is applied in the y-direction such that there is a slightly lower magnetic field at low y-values (towards the bottom of the bore as shown in Figure 2) and a slightly higher magnetic field at high y-values (towards the top of the bore as shown in Figure 2). This gradient is applied while the MR signal data is being measured. The gradient leads to slightly lower precession frequencies at the low y-values and slightly higher precession frequencies at the high y-values. The measured MR signal will comprise both the lower frequencies and the higher frequencies, as well as the frequency components between these extremes. The different frequencies in the MR signal can be differentiated and mapped to the different spatial locations along the y-direction.

Accordingly, the combination of phase encoding and frequency encoding provides in-plane measurements in the slice selected by the applied gradient. Varying the carrier frequency of the applied RF pulse enables different slices in a direction perpendicular to the plane of the slice (i.e. in a through-plane direction) to be measured. Therefore, these techniques enable three-dimensional information regarding a sample to be generated.

The magnetic fields of the nuclei induce a voltage in the radiofrequency coil 206, providing an MR signal. This output of the MR imaging process comprises raw data describing the frequencies of the MR signal. This raw data is k-space data. K-space data describes the spatial frequencies obtained from measurements of the sample. The k-space data can be represented as a 2D matrix of pixels with a frequency-encode axis and a phase-encode axis. Individual pixels within this matrix do not correspond to individual spatial locations of the subject. Instead, each pixel in the k-space matrix includes (partial) information relating to every spatial point that is measured in the sample. In order to obtain a spatial image from the k-space matrix, an inverse 2D Fourier transform can be applied to 'reconstruct' the image from the k-space data. This maps the measurements obtained, which are in terms of spatial frequencies, to measurements in terms of spatial locations within the sample. Where 3D k-space data is obtained and considered, the k-space matrix may be a 3D matrix of pixels with an additional dimension corresponding to the magnetic gradient direction. An inverse 3D Fourier transform may be applied to reconstruct a 3D image (i.e. 3D spatial information) from the k-space data.

While the above explanation has focused on a Cartesian sampling scheme in which the measurements are taken along a set of orthogonal axes, non-Cartesian sampling schemes are also possible. In non-Cartesian sampling schemes, measurements may be taken along radial or spiral trajectories, in which k-space data are obtained along overlapping 'spokes'. For example, 'stack-of-stars' sampling may be applied, which corresponds to a cylindrical coordinate system comprising radial spokes in-plane (e.g. in the x-y plane) and Cartesian sampling perpendicular to this plane (e.g. in the z-direction). In such radial sampling schemes, frequency encoding may be applied simultaneously in two directions (e.g. the x and y directions), rather than applying frequency encoding in one of these directions and phase encoding in the other of these directions.

Different types of tissue within the sample have different quantities of different atoms, which each have different proton densities and different spin-spin relaxation times and spin-lattice relaxation times. For example, fluids such as blood and spinal fluid have a higher proton density than bone due to containing a larger proportion of hydrogen atoms. As explained above, the RF signal applied can be tailored to cause a resonant response in hydrogen nuclei. The differences in the compositions of different body tissues enable these different types of tissue to be discriminated in the MR signal received. This enables generation of an image which shows different types of tissue with different contrast. For example, an image can be reconstructed which shows unhealthy tissue, such as a tumour, either lighter or darker than surrounding healthy tissue.

Generating an image depicting the prevalence of different types of tissue within a subject can be used to inform and/or guide treatments applied to the subject. For example, such an image may be used to guide radiotherapy treatment. The image may be used in treatment setup to determine or adjust a treatment plan, i.e. to determine or adjust an arrangement of angles and/or locations at which to apply radiotherapy. Alternatively, or in addition, the image may be generated and/or used during radiotherapy treatment to determine whether to halt, pause or adjust treatment.

As explained above, spatial encoding in MRI can be achieved by sampling the magnetization signal in combination with a linear magnetic field gradient. However, magnetic field variations, beyond the assumed linear gradient, can lead to incorrect spatial encoding of the signal, which results in geometrical distortion of the images produced. While magnetic field gradients may be optimized for linearity, small gradient non-linearities, and consequently small distortions, may still exist.

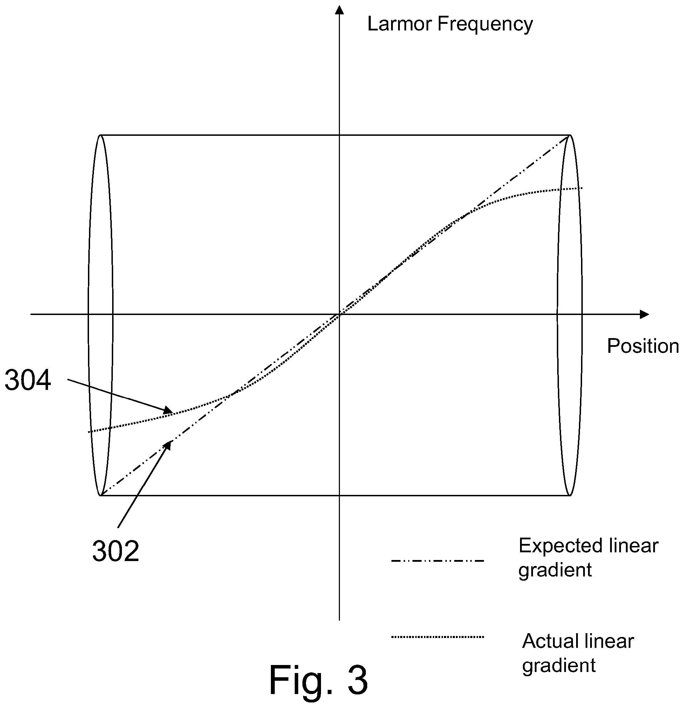

The non-linear components of the varying magnetic fields generated by the gradient coils are known as concomitant fields and are a consequence of Maxwell's equations, i.e. $\nabla \cdot \vec{B} = 0$. In radiotherapy, short imaging times are preferable to reduce the treatment time. As will be appreciated by the person skilled in the art, the need for fast MR imaging requires rapid slew rates (to create the required magnetic fields), which necessitate both shorter gradient coils and a relatively small number of coil turns. These constraints result in increased concomitant fields, making it difficult to maintain gradient linearity across the full range of the z-direction, as shown in Figure 3.

Figure 3 illustrates the magnetic field gradient in the z-direction in the bore of a radiotherapy device. This is illustrated in terms of Larmor frequency (which, as noted above, is proportional to magnetic field strength for any given nuclei) against position (which in this case is the z-position along the z-axis through the bore). A cylindrical overlay is also illustrated which represents the bore of the radiotherapy device.

The straight line 302 represents the expected Larmor frequency (or expected strength of the magnetic field) through the bore. As can be seen from Figure 3, this expected field strength has a linear gradient across the entire length of the bore. However, due to the engineering constraints noted above, the actual magnetic field (represented by line 304) produced by the gradient coils does not increase linearly. In other words, the actual magnetic field gradient across the length of the bore (the z-direction) is non-linear. Whilst not shown in this image, similar non-linear effects are found in the x-direction and y-direction due to similar constraints in the x-direction and y-direction gradient coils.

An effect of using non-linear magnetic field gradients in MR imaging is that the resulting MR image will exhibit geometrical distortion. For example, for an MR image slice acquired in the transverse orientation, the non-linearity in the x-direction and y-direction magnetic field gradients results in geometrical distortion in the plane of the image slice itself (i.e. the x-y plane). This is otherwise known as in-plane distortion. The non-linearity in the z-direction magnetic field gradient results in geometrical distortion through the plane of the slice (i.e. distortion in the z-direction). This is otherwise known as through-plane distortion. While described above for transverse MR image slices, similar distortions impact slices acquired in other orientations, such as the coronal or sagittal orientations.

In a clinical workflow, a series of 2D cine images may be obtained for the purposes of motion management. These may be distortion corrected both in-plane and through-plane. This distortion correction may be performed, for example, using a spherical harmonics mathematical model of the known non-linear gradient field components.

For the in-plane method, reconstructed motion-management images can be corrected using a 2D transformation calculated from the spherical harmonics model. The in-plane correction is highly accurate over the entire slice with only minor residual distortions remaining at the edge of the field of view.

The effect of the through-plane distortion is that 2D MR image slices are acquired in a curved plane instead of a flat plane. For the through-plane method, the spherical harmonics model can be used during the selection of a particular slice to offset the requested stack position. The offset is calculated such that the centre of the acquired motion-management image (i.e. center of the tracking structure) is warped in the through-plane to the position requested in the through-plane directions. In other words, it is known that the non-linearity of the gradient will cause a series of curved planes to be acquired, rather than a series of flat planes. To account for this, the amount of curvature that will result from the non-linearity is calculated and used to adjust which slice is allocated against a particular location along the z-axis, rather than just using the slice that would apply based on a naive assumption of there being no curvature. The determination of which slice to select is made such that the centre of the curved slice is in the requested location along the z-axis, even if the curvature of the slice means that the data acquired away from the centre of the curved slice will be from a different location along the z-axis to that requested and to that of the centre of the curved slice.

The approach explained above enables through-plane distortion to be corrected at the slice centre. However, away from the slice centre, i.e. away from the isocentre, through-plane distortion remains. To account for through-plane distortion over the whole slice, data from adjacent places would be needed. However, such data from adjacent planes are not typically acquired in order to enable fast cine imaging. In radiotherapy, particular regions of a subject anatomy can be identified, specified or delineated as contours. Contours may include one or more anatomical features of a subject, e.g. one or more targets (tumours) and/or one or more OARs. A contour may be defined in a structure set that describes the geometry of the associated anatomical feature, e.g. its outline or maximum spatial extent. The contour may be defined by a clinician and/or one or more computer programs based on images of the anatomy of the subject. Contours may be used to determine a treatment plan, e.g. to set beam angles, dose, etc to be used during a radiotherapy treatment.

As described above, it is known that acquired MR images will exhibit distortion. The expected distortion can be determined from the known, measured or calculated non-linearity of the magnetic field. This expected distortion may be applied to contours such that they exhibit the same distortion as acquired images. For example, the distortion applied to the contours may comprise the residual distortion remaining after the above-described in-plane distortion correction and through-plane distortion correction at the centre of the slice. In other words, the contours may be distorted such that they exhibit through-plane distortion away from the slice centre/isocentre. With the contour exhibiting a corresponding distortion to that of the MR images, more accurate target visualisation and motion management is enabled since the contour and the MR images have a corresponding geometrical structure, i.e. their underlying geometry corresponds.

As part of verification, the display accuracy of contours may be tested, for example using a motion-monitoring algorithm. A contour (distorted as described above) may be compared to the corresponding anatomical structure in an MR image. Since both of these exhibit through-plane distortion (at least away from the slice centre), the contour and the corresponding anatomical structure should have corresponding shapes. In other words, the contour overlaid on the MR image should outline the corresponding anatomical structure such that it follows the edge of the corresponding anatomical structure. This contour display accuracy test enables verification that the contour has been correctly processed and exhibits a high geometrical correspondence with the underlying subject anatomy. It can increase the accuracy and reliability of imaging processing and radiotherapy control through verifying that distortions have been accounted for in a consistent way.

The present disclosure provides techniques for efficiently evaluating the effect of through-plane geometrical distortion on displayed structure sets in regions of relatively high magnetic gradient non-linearity.

In radiotherapy, commissioning and device acceptance testing (DAT) are required to ensure that an installed radiotherapy device is working as expected. In addition, routine quality assurance (QA) is required to ensure that the device meets the expected tolerances. For conducting such testing, a phantom may be used. A phantom is a substitute for a subject's body or a part of a subject's body. In other words, a beam of radiation may be delivered to a phantom as part of testing to ensure that the beam of radiation can be safely and accurately delivered to a subject, with parameters of the radiotherapy device and the radiation delivery being as expected or intended within given tolerances. Imaging may be performed with a phantom in the intended location of a subject in order to verify whether the imaging meets accuracy and performance requirements. A phantom as described herein may be considered to be a substitute for or a physical model of a subject's body or a part of a subject's body. The phantom may not be a subject or a patient or a human or animal or a part thereof.

A phantom can be used in the above-mentioned contour display accuracy testing to further verify the safety and accuracy of radiotherapy for a subject. In this context, a contour may describe a subset of the geometry of the phantom. For example, a contour may describe an outline or maximum spatial extent of the phantom for a specified plane (i.e. in a specified orientation). A defined contour of the phantom can compared to a distorted MR image of the phantom. A defined contour of the phantom can be distorted and compared to a distorted MR image of the phantom.

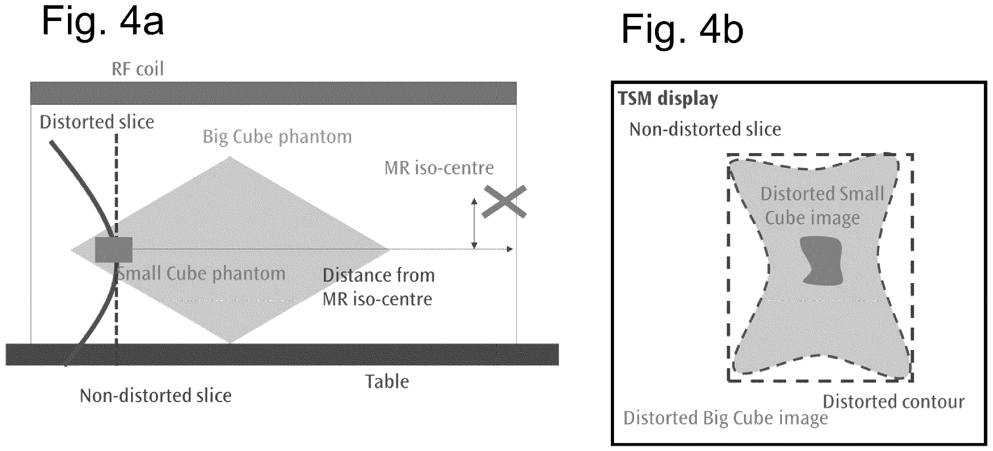

By way of example, one or more cube-shaped MR-visible phantoms may be used to verify that contours are accurately displayed, in in-plane and through-plane directions, over the underlying structure of the phantom. For example, a first (small) cube may be positioned inside a second (large) cube on the patient support surface 114 of a radiotherapy device. The cube(s) may be positioned away from the (MR) isocentre, i.e. in the distorted zone. A slice may be obtained at a 45° angle to the cube edges (or at a different non-perpendicular and non-parallel angle). For example, a slice may be obtained in the x-z, y-z or y-z plane and the cube may be oriented at a 45° angle to the relevant plane (or at a different non-perpendicular and non-parallel angle). This arrangement is depicted in Figure 4a.

Due to the non-linearity of the magnetic field described above, when a slice in a flat plane is requested, the signal produced is actually from a distorted (curved) slice. If there was no through-plane distortion, the representation of the phantom in the acquired slice would be rectangular. However, because the slice is curved, the representation of the phantom in the acquired slice is distorted due to some of the data being acquired closer to the corner of the phantom where its width is smaller. This is depicted in Figure 4b. The distorted big cube image has curved portions deviating from a rectangle. A known or measured contour of the phantom can be distorted to exhibit the same distortion it is expected that the acquired MR image will exhibit. The curved dotted line surrounding the distorted big cube image depicts this distorted contour. The distorted contour closely matches the shape of the distorted big cube image. This indicates that the distortion correction was successfully and consistently applied since both the contour and the image exhibit corresponding distortions such that their overlaid shapes match.

The contour display accuracy testing using a phantom as described above may be difficult to perform for some orientations. A cuboid phantom may be positioned with one of its faces resting on the horizontal patient support surface 114 with side edges pointing in the x-direction and the z-direction, i.e. with its side faces oriented at 45° to the x-axis and the z-axis. In this arrangement, the width of the cube varies along the x-axis and the z-axis because the cube narrows along these axes as the corners of the cube are approached. However, the width/extent of the cube is constant in the y-direction. As such, a slice which is curved with respect to the y-axis will acquire data from locations of the cube at different positions along the y-axis (i.e. at different 'heights' of the cube), but because the extent of the cube is constant in this direction, the representation of the cube in the distorted image slice may still be a rectangle rather than a distorted rectangle.

The above discussion is provided in terms of the x, y, and z axes of the radiotherapy device. For a typical arrangement in which a subject is lying on their back on the patient support surface 114, the same discussion can be given in terms of the transverse, sagittal and coronal planes as follows.

A transverse slice may be obtained in the x-y plane. The through-plane direction for this slice is in the z-direction. The width of the phantom arranged as described above varies in the z-direction such that contour display accuracy due to through-plane distortion can be tested using this arrangement.

A sagittal slice may be obtained in the y-z plane. The through-plane direction for this slice is in the x-direction. The width of the phantom arranged as described above varies in the x-direction such that contour display accuracy due to through-plane distortion can be tested using this arrangement.

A coronal slice may be obtained in the x-z plane. The through-plane direction for this slice is in the y-direction. The width of the phantom arranged as described above does not vary in the y-direction such that contour display accuracy due to through-plane distortion cannot be tested using this arrangement.

One possible approach to address this is to re-orient the cube such that its width does vary in the y-direction. In particular, the cube may be balanced on the patient support surface 114 such that only an edge of the cube contacts the patient support surface 114, rather than a face of the cube contacting the patient support surface 114. The cube may be supported on its edge using one or more objects or accessories such as pieces of foam. However, it will be appreciated that this is potentially an unstable arrangement. This is especially the case because the phantom may be filled with liquid, e.g. water, such that water may leak out with the phantom balanced in this orientation. It also requires substantial manual intervention and is difficult to reproduce reliably.

The present disclosure provides improved phantoms suitable for improved contour overlay testing in view of through-plane geometrical distortion.

As described herein a phantom comprises an outer surface tapered along at least one axis of the phantom towards a tapered end. In other words, the outer surface may be tapered along the at least one axis between a base and the tapered end. In some implementations, the outer surface may taper, i.e. progressively narrow, along an entire length of the phantom along the at least one axis. In other words, the outer surface may taper or narrow towards a vertex or apex or peak. For example, the outer surface may have a pyramidal shape. The outer surface may comprise at least one pyramidal structure. The pyramid may comprise a square base, and may comprise four triangular faces between the square base and the vertex. In some examples, the outer surface may taper, i.e. progressively narrow, along only part of the length of the phantom along the at least one axis. For example, the outer surface may have a truncated pyramid shape. The pyramid may have a base comprising any regular or irregular polygon or other shape. The base of the phantom or outer surface may comprise a part of the outer surface suitable for disposing or fixing on the patient support surface 114. The outer surface of the pyramid may be conical or may have the shape of a wedge, cupola or frustrum.

The phantom comprises a fixation mechanism. The fixation mechanism is configured to fix the phantom on a patient support surface 114 of a radiotherapy device such that the tapered end can selectively be aligned at different respective times with each of three perpendicular axes of the radiotherapy device. These three axes may comprise: a first axis oriented into-out of a bore of the radiotherapy device; a second axis oriented from a left side to a right side of the radiotherapy device; and a third axis oriented from a bottom to a top of the radiotherapy device.

The combination of phantom geometry and fixation mechanism described above enables improved evaluation of contour display accuracy using the phantom. In particular, being able to orient a tapered end to align with each of the three axes of the radiotherapy device enables the effects of through-plane distortion to be evaluated for all of transverse, sagittal and coronal planes. The fixation mechanism enables this to be performed reproducibly, accurately and reliably.

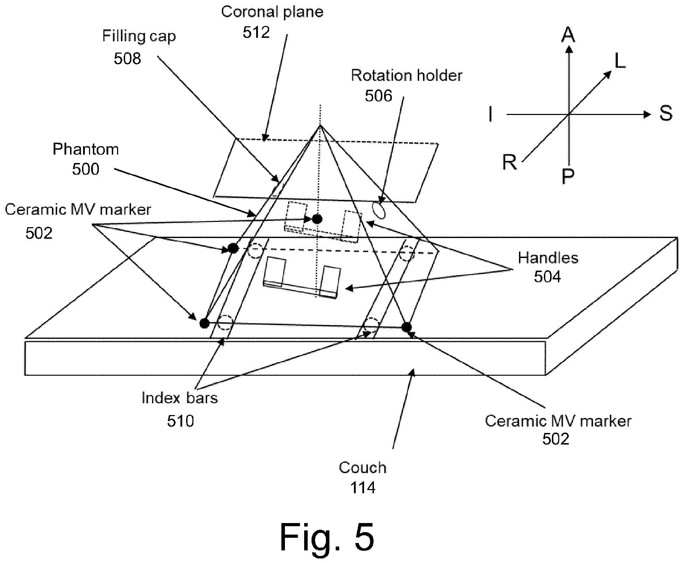

A first implementation of a phantom 500 according to the present disclosure is depicted in Figure 5. The phantom is depicted fixed to the patient support surface or couch 114. A set of axes is provided in Figure 5, which provides the anatomical planes of a subject lying on their back on the patient support surface 114 with their head toward the right side of the figure. The S-I axis is the superior-inferior (head to feet) axis. The A-P axis is the anterior-posterior (front to back) axis. The L-R axis is the left-right or lateral-medial axis. In the coordinate system of a radiotherapy device as described herein, the S-I axis corresponds to the z-axis, the A-P axis corresponds to the y-axis and the L-R axis corresponds to the x-axis.

By way of example, the phantom 500 depicted in Figure 5 has a pyramidal shape, and in particular a square pyramid shape, though the present disclosure is not limited thereto. In Figure 5, the square surface (which may be referred to as the base of the pyramid for ease of explanation), is disposed on the patient support surface 114.

The phantom 500 comprises a fixation mechanism configured to fix the phantom 500 on or to the patient support surface 114, for example by interfacing or coupling with a second fixation mechanism which is part of or attached to the patient couch 114. For example, the phantom 500 may comprise indents or holes in its base (i.e. in the square surface of the phantom 500). The second fixation mechanism may comprise index bars 510. The index bars 510 may comprise protrusions or nodules. The indents may be shaped, sized and positioned so as to receive the protrusions of the index bars 510. This may enable the phantom 500 to be fixed on the patient support surface 114 quickly, efficiently and reproducibly. It will be appreciated that other fixation mechanisms, accessories or mountings may be used alternatively or in addition to those described above, for example using one or more of bolts, threaded holes, clamps and sliding rails.

The phantom 500 may comprise one or more MV markers 502. These are made from a material that is detectable via MV (or kV) imaging. This material may be a dense material adequate for blocking X-rays such that it shows up on an MV/kV image relative to its surroundings. The material may be a ceramic. The MV markers 502 may be used to determine or verify the position and/or orientation of the phantom 500 using MV/kV imaging. For example, the phantom 500 depicted in Figure 5 has three MV markers 502 each at a respective corner of the square surface (base), and one MV marker 500 located directly above the centre of the square surface and directly below the tip of the pyramid. It will be appreciated that other arrangements of MV markers 502 are possible so long as they enable different positions and orientations of the phantom 500 to be distinguished.

The phantom 500 may be made of an MR-compatible plastic. The phantom 500 may comprise one or more materials that are detectable via MR imaging. This material may be a material comprising a particular chemical makeup that is readily identifiable via MR imaging. The material may comprise hydrogen atoms or molecules comprising hydrogen atoms. The material may comprise water or MR oil. The phantom 500 may comprise a cavity or container for retaining the water or MR oil. In other words, the phantom 500 may be filled with the water or MR oil. The phantom 500 may comprise a filling hole for introducing water or MR oil to the interior of the phantom 500 and a filling cap 508 for sealing this filling hole and thereby preventing the water or MR oil from leaking (e.g. when the orientation of the phantom 500 is adjusted).

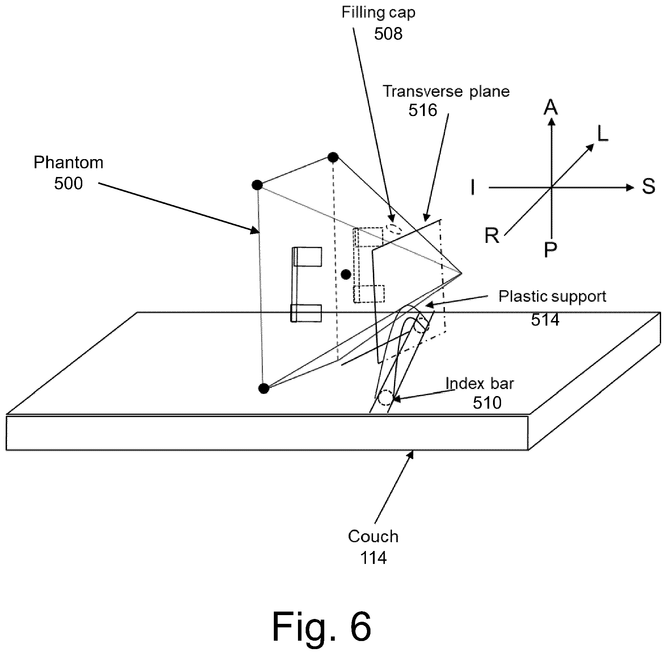

The phantom 500 may comprise a rotation holder 506, which will be discussed further in relation to Figure 6. The phantom 500 may also comprise handles 504 to enable lifting of the phantom 500 by a user.

Figure 5 depicts a coronal plane 512. The phantom 500 oriented and fixed as depicted in Figure 5 may be suitable for measuring through-plane distortion in a coronal slice. This is because the phantom 500 tapers or narrows towards a tapered end in a direction perpendicular to the coronal plane 512. As such, imaging data that is acquired from spatial locations slightly higher or lower than intended (due to magnetic gradient non-linearity) will image a narrower or wider part of the phantom 500 such that a through-plane distorted image of the phantom 500 will be acquired.

In order to measure through-plane distortion for different planes, the phantom 500 may be reoriented. Figure 6 depicts the phantom 500 reoriented such that its tip or tapered end is oriented parallel to the superior-inferior axis. Figure 6 also depicts a transverse plane 516. The phantom 500 oriented and fixed as depicted in Figure 6 may be suitable for measuring though-plane distortion in a transverse slice. This is because the phantom 500 tapers or narrows towards a tapered end in a direction perpendicular to the transverse plane 516. As such, imaging data that is acquired from spatial locations slightly to the left or right of Figure 6 than intended (due to magnetic gradient non-linearity) will image a narrower or wider part of the phantom 500 such that a through-plane distorted image of the phantom 500 will be acquired.

The fixation mechanism of the phantom 500 may comprise a support 514, which may be made from plastic. The support 514 may couple or interface with the rotation holder 506 and with one or more of the index bars 510. For example, the support 514 may comprise a bar, for example a curved bar, with holes or indents therein. The holes may be sized, shaped and positioned to receive protrusions or nodules of the index bar 510. In addition, the support 514 may have a shape or protrusion that couples or interfaces with the rotation holder 506 of the phantom 500. For example, the rotation holder may comprise one or more holes or slots for receiving the support 514. These components may therefore enable fixation of the phantom 500 on the patient support surface or couch 114 in a desired orientation in a reproducible, accurate and reliable way, as well as enabling this to be performed quickly and efficiently. As depicted in Figure 6, this desired orientation may be with the tip of the phantom 500 oriented along the superior-inferior axis and the square surface (base) of the phantom 500 oriented perpendicular to the superior-inferior axis. It will be appreciated that other fixation mechanisms, accessories or mountings may be used alternatively or in addition to those described above, for example using one or more of bolts, threaded holes, clamps and sliding rails.

While not depicted, it will be appreciated that the phantom 500 can also be reoriented such that its tip or tapered end is oriented parallel to the left-right axis. In other words, relative to Figure 6, the phantom 500 may be rotated 90° about the anterior-posterior axis. The phantom 500 oriented in this way may be suitable for measuring though-plane distortion in a sagittal slice. This is because the phantom 500 tapers or narrows towards a tapered end in a direction perpendicular to the sagittal plane. As such, imaging data that is acquired from spatial locations slightly displaced along the left-right axis relative to the spatial locations that were intended (due to magnetic gradient non-linearity), will image a narrower or wider part of the phantom 500 such that a through-plane distorted image of the phantom 500 will be acquired.

The above-described fixation mechanisms may also be used for orienting and fixing the phantom 500 for measuring the through-plane distortion of the sagittal slice. In particular, the support 514 may couple or interface with the rotation holder 506 and with one or more of the index bars 510 in a similar manner to that described in relation to Figures 5 and 6.

Alternatively, the phantom 500 may not be rotated in order to evaluate through-plane distortion in transverse and sagittal planes. While the outer surface of the phantom 500 as depicted in Figure 5 varies most significantly in the anterior-posterior direction, the outer surface also varies to a lesser degree in the superior-inferior direction and the left-right direction. Therefore, through-plane distortion and contour display accuracy may also be evaluated for planes perpendicular to these directions with the phantom 500 oriented as depicted in Figure 5. However, since the relevant gradients for these directions are less significant, it may be more difficult to evaluate the through-plane distortion and contour display accuracy in an accurate manner without the reorientation of the phantom 500 described above.

While one implementation of a phantom has been described above in relation to Figures 5 and 6, other implementations of phantoms having different shapes are considered herein and are within the scope of the present disclosure. These phantoms narrow along at least one of their axes and comprise a fixation mechanism enabling the phantom to be fixed on a patient support surface 114 of a radiotherapy device such that the narrowed end of the phantom can selectably be aligned with all three axes of the radiotherapy device. For example, the narrowed end may be aligned with a first of these axes, reoriented to be aligned with a second of these axes, and reoriented again to be aligned with the third of these axes.

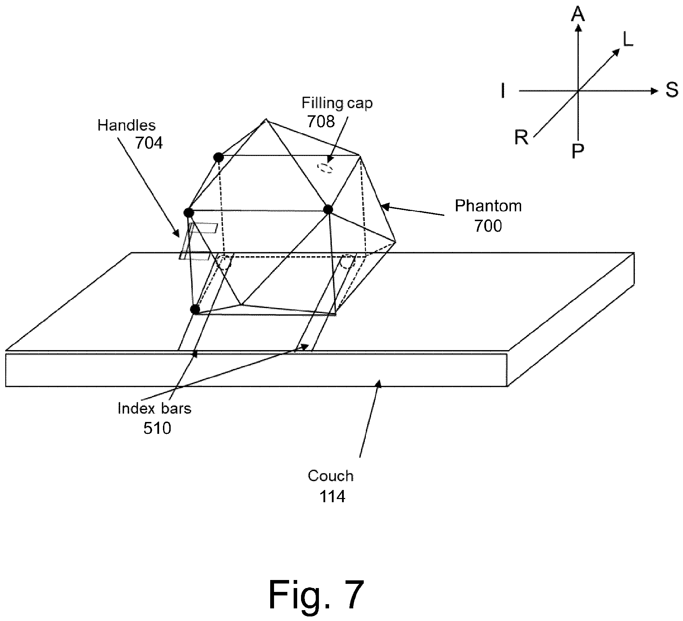

Figure 7 depicts an alternative implementation of a phantom 700. The phantom 700 may be similar to the phantom 500 unless mentioned otherwise and therefore unnecessary repetition will be omitted. The shape of phantom 700 may be that of a cube with a respective square-based pyramidal feature on each of three (virtual) faces of the cube. In other words, three of the (virtual) faces of the cube may be distorted outwards into respective points, such that the respective (virtual) face of the cube is the (virtual) base of respective pyramid. In other words, the phantom 700 may have a three-dimensional polygonal shape with three square faces and twelve triangular faces comprising three sets of four triangular faces. Each triangular set of four triangular faces may meet at a point, i.e. to form a pyramidal feature or structure. Each of the points may be perpendicular to each of the other points.

As can be seen from Figure 7, the first point of a first of the pyramidal features is aligned with the superior-inferior axis. The second point of a second of the pyramidal features is aligned with the anterior-posterior axis. The third portion of a third of the pyramidal features is aligned with the left-right axis. Accordingly, through-plane distortion may be measured for slices taken in each of the transverse, coronal and sagittal planes with the phantom 700 oriented as depicted in Figure 7, i.e. without reorienting the phantom 700 between these measurements. This can be advantageous in that it may reduce manual intervention required to reorient the phantom 700 and may speed up the measurements. For example, the through plane distortion for all three planes may be measured as part of the same automated image acquisition sequence. In some implementations, the fixation mechanism of the phantom 700 may only be configured to fix the phantom 700 on the patient support surface 114 with one orientation. On the other hand, the geometry of the phantom 700 may mean that the through-plane distortion of images may only be measured for particular slices at particular distances along the three axes and/or the more complex geometry of phantom 700 may make evaluations of the distortion to its shape more complicated.

The phantom 700 may comprise a filling cap 708 and handles 704 similar to those described above in relation to Figure 5. The phantom 700 may comprise a fixation mechanism, in a similar manner to that described above, for interfacing or coupling with a second fixation mechanism of the patient support surface or couch 114 of a radiotherapy device, for example the index bars 510.

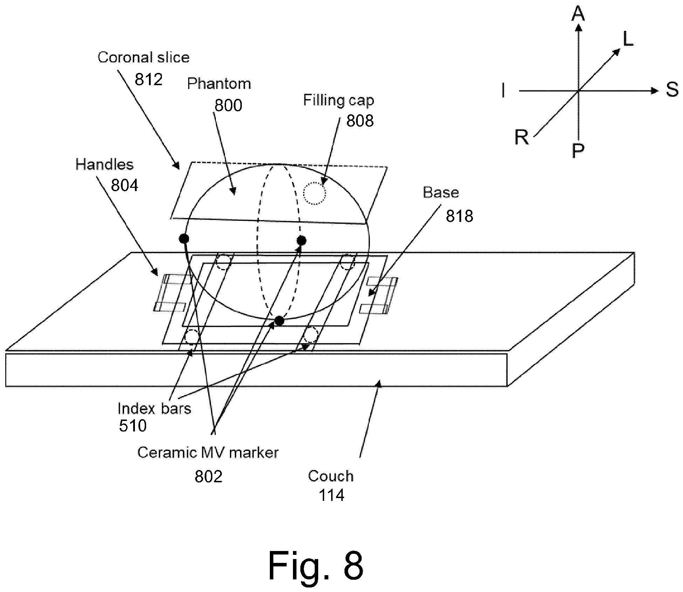

Figure 8 depicts an alternative implementation of a phantom 800. The phantom 800 may be similar to the phantoms 500 and 700 unless mentioned otherwise and therefore unnecessary repetition will be omitted. The phantom 800 may be spherical or spheroid in shape. This geometry enables through-plane distortion to be measured in any arbitrary plane (including for the transverse, sagittal and coronal planes discussed above). However, the amount of through-plane distortion seen may be smaller than that seen for a pyramidal shape as the through-plane gradient of the shape is relatively reduced.

The phantom 800 may not need to be rotated or reoriented in order to measure through-plane distortion in different planes. The phantom 800 may be disposed on or fixed to a base 818, which may interface or couple with the index bars 510 of the patient support surface or couch 114. In some implementations, the fixation mechanism of the phantom 800 may only be configured to fix the phantom 700 on the patient support surface 114 with one orientation. The phantom 800 may comprise MV markers 802, handles 804 and/or filling cap 808 similar to those described above. Figure 8 also depicts a coronal plane or slice 812.

Accordingly, the current disclosure provides a phantom with an outer surface which is shaped or rotatable such that the geometry of the outer surface varies along three perpendicular axes of the radiotherapy device when the phantom is disposed on a patient support surface 114 of the radiotherapy device.

The phantoms as described herein may comprise one or more dosimeters, such as electronic dosimeters, thermoluminescent dosimeters, dosimetric films, alanine pellets, gel dosimeters, etc. The one or more dosimeters may be disposed, embedded or contained within the phantom, or disposed on the surface of the phantom. In some examples, the phantom may comprise one or more accessible compartments each for receiving respective dosimeters. Inclusion of multiple dosimeters may enable a spatially resolved radiation dose to be determined such to provide information on what doses different parts of a subject can be expected to receive. The dosimeters may be disposed in a regular pattern such as in a cubic or helical grid. Alternatively, the dosimeters may be disposed in a random pattern. Each of the one or more dosimeters may be configured to measure respective measured values of dose, for example with the phantom disposed on the patient couch while a radiotherapy treatment is delivered according to a treatment plan.

The present disclosure provides improved techniques for evaluating contour display accuracy in view of through-plane distortion. These techniques may be performed using one or more of the phantoms described herein. A contour may comprise a geometrical structure comprising a particular subset of patient anatomy (e.g. a tumour or OAR) or of phantom geometry. Corresponding distortion may be applied to the contour as is expected or calculated to be exhibited in through-plane distorted MR images. The present disclosure enables comparison of the distorted or undistorted contour to corresponding features of a through-plane distorted MR image in order to evaluate the accuracy of image processing and contour display.

Accordingly, the present disclosure provides a method for evaluating contour display accuracy for radiotherapy. The method comprises obtaining, by a control device, a through-plane distorted MR image comprising a representation of a phantom disposed on a patient support surface of a radiotherapy device. The method further comprises determining, by the control device, a difference between the representation of the phantom and a reference contour of the phantom. This enables improved determination of the accuracy of imaging and image processing in radiotherapy. The method may comprise additional steps/features as explained herein.

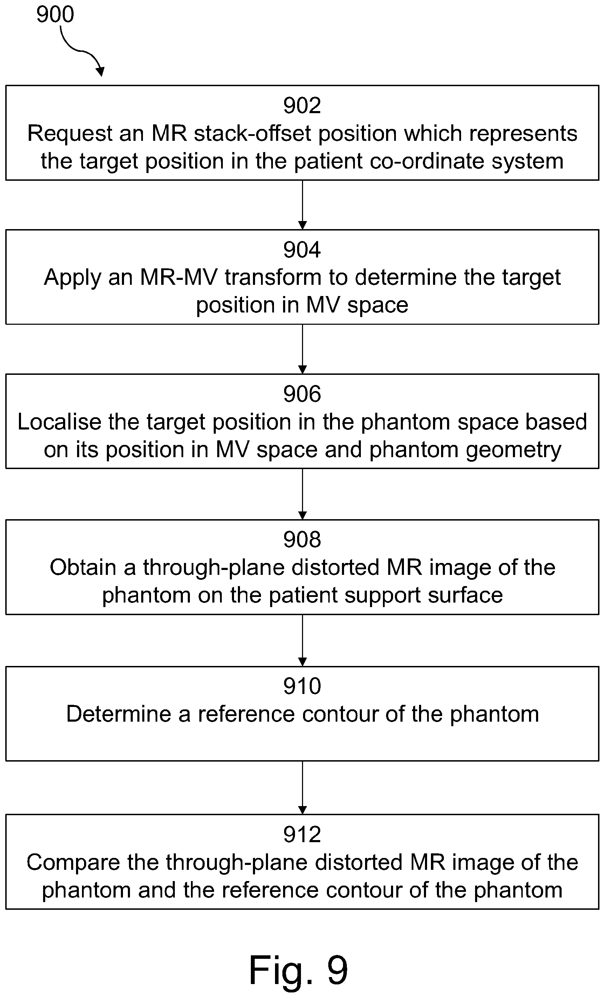

Figure 9 depicts an example method 900 for evaluating contour display accuracy for radiotherapy. The method may be performed by a control device as described herein. The method may be performed using a phantom as described herein in order to test the method and associated processes for performance with a patient positioned in place of the phantom. The same method is applicable to both the phantom and patient anatomy such that references to these may be made interchangeably.

In a step 902, the method may comprise requesting an MR stack-offset position which represents or corresponds to the target position in the patient co-ordinate system. This MR stack-offset position may be referred to as $MR_{Pos} = (x_{MR}, y_{MR}, z_{MR})^T$.

In a step 904, an MR-MV transform may be applied to determine the target position in MV space. The MR-MV transform may be referred to as $M_{MR}^{MV}$ and the target position in MV space may be referred to as $MV_{Pos} = (x_{MV}, y_{MV}, z_{MV})^T$, where

$$MV_{Pos} = M_{MR}^{MV} MR_{pos}$$

In a step 906, the target position may be localised in the phantom space $G_{pos} = (x_G, y_G, z_G)^T$ based on its position in MV space and the known relationship to the MV markers and phantom geometry $M_{MV}^G$, where

$$G_{pos} = M_{MV}^G MV_{pos}$$

In a step 908, a through-plane distorted MR image $C_A$ of the phantom on the patient support surface 114 may be obtained. This image exhibits through-plane distortion due to the non-linearity of the magnetic field gradient, as explained above. The image may have been corrected for in-plane distortion and for through-plane distortion at its centre. The image may be obtained such that the centre of the image coincides with the target, for example using the above-mentioned coordinate definitions and transformations. In some examples, $C_A$ may be determined using segmentation methods, to isolate particular anatomical/phantom features or regions, or may be estimated by warping the expected 3D distortion corrected contour in the through-plane based on distortion vector fields calculated from a spherical harmonics magnetic field model.

In a step 910, a reference contour of the phantom may be determined. The reference contour may be determined by extracting the desired slice from the phantom geometry. In some implementations, the reference contour may be the ground-truth contour $C_G$. This may be the slice with centroid $G_{pos}$ extracted from the known phantom geometry, which may itself be determined from design drawings or from a CT scan of the phantom. In other words, the ground-truth contour may correspond to the actual physical shape and dimensions of the phantom for the extracted slice, i.e. without any distortion. The reference contour and the through-plane distorted MR image may be described as being for corresponding (i.e. the same) subsets and orientations of the patient anatomy/phantom shape. In other words, the centroid location and plane orientation of the reference contour and image may generally correspond, or at least may differ only due to the distortion described herein.

Alternatively, in step 910 the reference contour may be a distortion corrected contour $C_M$, i.e. a 3D distortion corrected contour. This may be generated by applying through-plane distortion correction to the obtained through-plane distorted MR image $C_A$. In this case, the difference between $C_M$ and $C_G$ is associated with residual distortion errors after correction. Alternatively, in step 910 the reference contour may be a warped distortion corrected contour $C_{MWarp}$, i.e. a warped 3D distortion corrected contour. This may be generated by warping the distortion corrected contour $C_M$ such that it (re-)exhibits through-plane distortion. In this test, the effect of through-plane distortion ($C_{MWarp} - C_A$) represents a performance measure of the visualisation display.

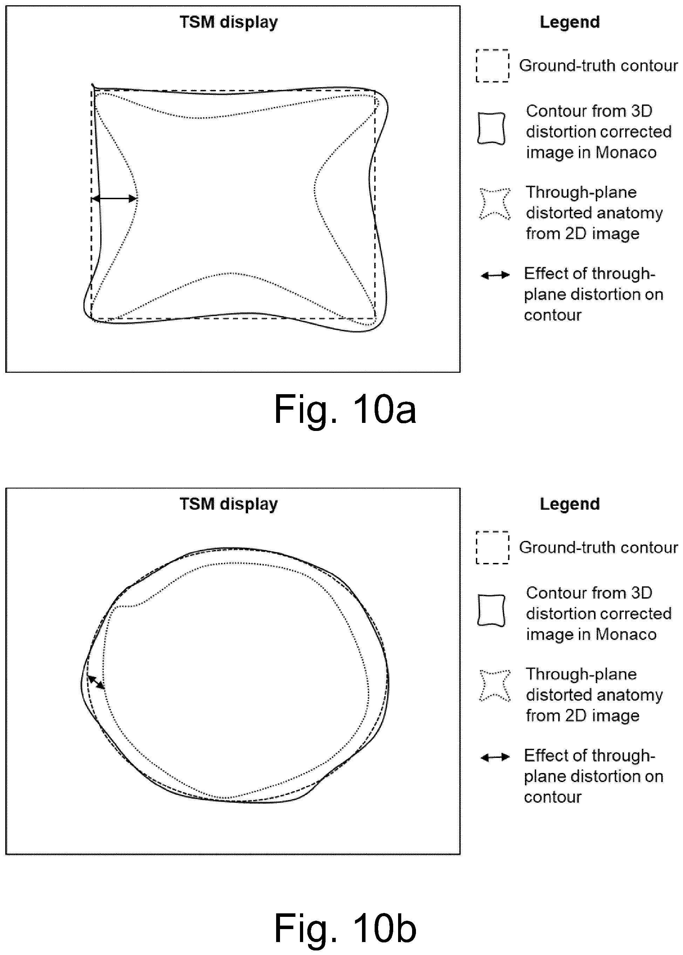

In a step 912, the through-plane distorted MR image $C_A$ of the phantom may be compared to the reference contour of the phantom. For example, a difference may be determined between the through-plane distorted MR image and the reference contour (as further explained below in relation to Figures 10a and 10b). Where the reference contour is the ground-truth contour, this difference may be $C_G - C_A$. Where the reference contour is the distortion corrected contour, this difference may be $C_M - C_A$. This provides an indication of the residual distortion error remaining after correction. Where the reference contour is the warped distortion corrected contour, this difference may be $C_{MWarp} - C_A$. This provides an indication of a performance measure of visualisation display/the processing steps applied. The user may manually measure the difference, or the difference may be calculated using QA software. The through-plane distorted image and the reference contour may be displayed on a display screen to provide a visual indication of the differences between them.

Figures 10a and 10b depict example implementations of comparisons between through-plane distorted images and reference contours. These may be depictions of motion management software on a display apparatus. For example, these may comprise part of the display of a Treatment Session Manager (TSM). Figure 10a may comprise an image and contours of a pyramidal phantom 500. Figure 10b may comprise an image and contours of a spherical or spheroid phantom 800.

Referring to Figure 10a, the ground-truth contour (dashed line) can be seen to be rectangular, i.e. to exhibit no distortion. The through-plane distorted MR image (dotted line), i.e. the representation of the phantom from the through-plane distorted MR image, is distorted due to the non-linearity of the magnetic gradient and the geometry/orientation of the phantom with respect to this gradient (as described above). Accordingly, the through-plane distorted MR image is distorted with respect to the ground-truth contour, as depicted using the double-headed arrow showing the effect of through-plane distortion. Figure 10a also depicts (solid line) a distortion corrected contour (i.e. $C_M$ as referred to in relation to Figure 9), which may be obtained from treatment planning software such as Monaco. Relative to the through-plane distorted MR image, the distortion corrected contour is closer to the ground-truth image. However, residual 'errors' remain.

The difference between representation of the phantom from the through-plane distorted MR image and the reference contour may be determined in various ways. In some implementations, respective separations between each of a set of points on the reference contour and a corresponding set of points on the representation of the phantom from the through-plane distorted MR image may be determined. In some implementations, a first separation between the centre of the slice and each of a set of points on the reference contour and a second separation between the centre of the slice and each of a corresponding set of points on the representation of the phantom from the through-plane distorted MR image may be compared (i.e. subtracted to find the difference between them). In other words, the separations may be determined for a series of spokes starting at the centre and radiating outwards. The separations, and the difference between them, may be vector displacements or absolute distances. In some implementations, the difference may be determined by determining the maximum distance or displacement between the representation of the phantom and the reference contour, for example the maximum distance or displacement in directions along spokes passing through the centre of the slice. In some examples, a difference in area or circumference between the representation of the phantom and the reference contour may be determined. In some implementations, the separation or displacement between corners of the reference contour and representation may be determined. In some implementations, the separation or displacement between locations mid-way between corners of the reference contour and representation may be determined (e.g. at the point mid-way between the upper and lower left corners as depicted in Figure 10a). It will be appreciated that any suitable means of comparing the relevant shapes or outlines may be used for these purposes without loss of generality.

Referring to Figure 10b, the ground-truth contour (dashed line) can be seen to be a circle or oval corresponding to the spheroid shape of the phantom 800. The through-plane distorted MR image (dotted line) is distorted due to the non-linearity of the magnetic gradient and the geometry/orientation of the phantom with respect to this gradient (as described above). Accordingly, the through-plane distorted MR image is distorted with respect to the ground-truth contour, as depicted using the double-headed arrow showing the effect of through-plane distortion. Figure 10b also depicts (solid line) a distortion corrected contour (i.e. $C_M$ as referred to in relation to Figure 9), which may be obtained from treatment planning software such as Monaco. Relative to the through-plane distorted MR image, the distortion corrected contour is closer to the ground-truth image. However, residual 'errors' remain. The differences between the representation of the phantom from the through-plane distorted MR image and the reference contour may be determined in various ways as described above in relation to Figure 10a.

As will be appreciated, the representation of the phantom and the reference contour between which the difference is found may have the same centre or centroid. In other words, the centre of the reference contour may correspond to the centre of the representation of the phantom. However, since the through-plane distorted image is acquired in a curved plane, the spatial locations of the phantom based on which the representation of the phantom is derived will in general differ from the spatial locations of a reference contour (e.g. a ground truth contour corresponding to a flat plane).

While the methods disclosed herein are presented in a certain sequential order, this should not be taken to limit the methods to the orders presented. One or more of the method steps may be omitted or rearranged. The various steps may be performed in different orders. Various steps may be performed at the same time or substantially the same time. Herein, references to events occurring substantially at the same time may refer to events at least partially overlapping in time and/or events occurring at the same time within measurement uncertainties.

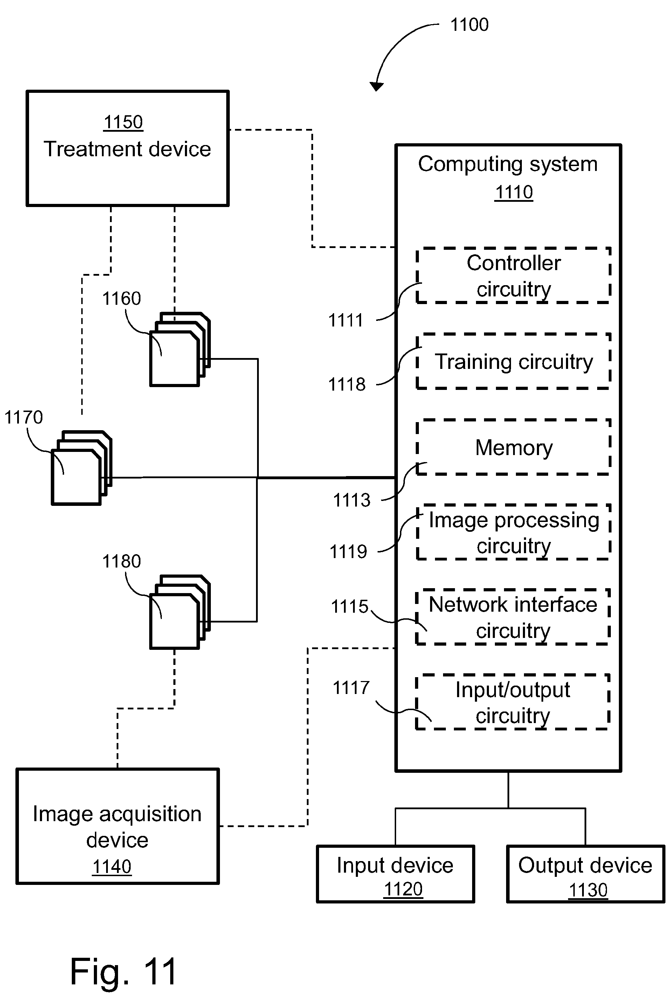

Figure 11 illustrates a block diagram of one implementation of a radiotherapy system 1100. The radiotherapy system 1100 comprises a computing system 1110 within which a set of instructions, for causing the computing system 1110 to perform any one or more of the methods discussed herein, may be executed. The computing system 1110 may also be referred to as a control device as used herein.

The computing system 1110 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 1110 includes controller circuitry 1111 and a memory 1113 (e.g., read-only memory (ROM), flash memory, dynamic random access memory (DRAM) such as synchronous DRAM (SDRAM) or Rambus DRAM (RDRAM), etc.). The memory 1113 may comprise a static memory (e.g., flash memory, static random access memory (SRAM), etc.), and/or a secondary memory (e.g., a data storage device), which communicate with each other via a bus (not shown).

Controller circuitry 1111 represents one or more general-purpose processors such as a microprocessor, central processing unit, accelerated processing units, or the like. More particularly, the controller circuitry 1111 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 1111 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 1111 is configured to execute the processing logic for performing the operations and steps discussed herein.

The computing system 1110 may further include a network interface circuitry 1115. The computing system 1110 may be communicatively coupled to an input device 1120 and/or an output device 1130, via input/output circuitry 1117. In some implementations, the input device 1120 and/or the output device 1130 may be elements of the computing system 1110. The input device 1120 may include an alphanumeric input device (e.g., a keyboard or touchscreen), a cursor control device (e.g., a mouse or touchscreen), an audio device such as a microphone, and/or a haptic input device. The output device 1130 may include an audio device such as a speaker, a video display unit (e.g., a liquid crystal display (LCD) or a cathode ray tube (CRT)), and/or a haptic output device. In some implementations, the input device 1120 and the output device 1130 may be provided as a single device, or as separate devices.

In some implementations, computing system 1110 includes training circuitry 1118. The training circuitry 1118 is configured to train a method of evaluating images and/or a method of detecting large deformations in images. For example, training circuitry 1118 may train a model for performing a method of evaluating images/a method of detecting large deformations in images. The model may comprise a deep neural network (DNN), such as a convolutional neural network (CNN) and/or recurrent neural network (RNN). Training circuitry 1118 may be configured to execute instructions to train a model that can be used to evaluate images/detect large deformations in images, as described herein. Training circuitry 1118 may be configured to access training data and/or testing data from memory 1113 or from a remote data source, for example via network interface circuitry 1115.

In some examples, training data and/or testing data may be obtained from an external component, such as image acquisition device 1140 and/or treatment device 1150. In some implementations, training circuitry 1118 may be used to update, verify and/or maintain a model for determining a difference between a representation of a phantom and a reference contour of the phantom as described herein.

In some implementations, the computing system 1110 may comprise image processing circuitry 1119. Image processing circuitry 1119 may be configured to process image data 1180 (e.g. images, or imaging data), such as medical images obtained from one or more imaging data sources, a treatment device 1150 and/or an image acquisition device 1140 as described herein. Image processing circuitry 1119 may be configured to process, or pre-process, image data. For example, image processing circuitry 1119 may convert received image data into a particular format, size, resolution or the like. In some implementations, image processing circuitry 1119 may be combined with controller circuitry 1111.

In some implementations, the radiotherapy system 1100 may further comprise an image acquisition device 1140 and/or a treatment device 1150, such as those disclosed herein (e.g. in relation to Figure 1). The image acquisition device 1140 and the treatment device 1150 may be provided as a single device. In some implementations, treatment device 1150 is configured to perform imaging, for example in addition to providing treatment and/or during treatment. The treatment device 1150 comprises the main radiation delivery components of the radiotherapy system described herein.

Image acquisition device 1140 may be configured to perform positron emission tomography (PET), computed tomography (CT), magnetic resonance imaging (MRI), etc.

Image acquisition device 1140 may be configured to output image data 1180, which may be accessed by computing system 1110. Treatment device 1150 may be configured to output treatment data 1160, which may be accessed by computing system 1110.

Computing system 1110 may be configured to access or obtain treatment data 1160, planning data 1170 and/or image data 1180. Treatment data 1160 may be obtained from an internal data source (e.g. from memory 1113) or from an external data source, such as treatment device 1150 or an external database. Planning data 1170 may be obtained from memory 1113 and/or from an external source, such as a planning database. Planning data 1170 may comprise information obtained from one or more of the image acquisition device 1140 and the treatment device 1150.

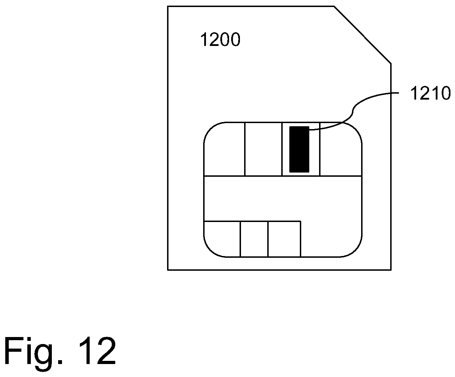

The various methods described above may be implemented by a computer program. The computer program may include computer code (e.g. instructions) 1210 arranged to instruct a computer to perform the functions of one or more of the various methods described above. The steps of the methods described above may be performed in any suitable order. The computer program and/or the code 1210 for performing such methods may be provided to an apparatus, such as a computer, on one or more computer readable media or, more generally, a computer program product 1200)), depicted in Figure 12. The computer readable media may be transitory or non-transitory. The one or more computer readable media 1200 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 1210 may also reside, completely or at least partially, within the memory 1113 and/or within the controller circuitry 1111 during execution thereof by the computing system 1110, the memory 1113 and the controller circuitry 1111 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining," "identifying," "obtaining," "detecting," "generating," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

The disclosure comprises the following items:

1. A method for evaluating contour display accuracy for radiotherapy, the method comprising:

obtaining, by a control device, a through-plane distorted MR image comprising a representation of a phantom disposed on a patient support surface of a radiotherapy device; and determining, by the control device, a difference between the representation of the phantom and a reference contour of the phantom.

2. A method according to item 1, wherein the reference contour of the phantom comprises a ground truth contour of the phantom which is not distorted.

3. A method according to item 1, wherein the reference contour of the phantom comprises a distortion-corrected contour of the phantom.

4. A method according to item 1, wherein the reference contour of the phantom comprises a distortion-corrected contour of the phantom which has been warped such that it exhibits through-plane distortion.

5. A method according to any preceding item, further comprising:

rotating the phantom about a first axis such that the phantom is disposed on the patient support surface in a first rotated position; obtaining, by the control device, a second through-plane distorted MR image comprising a second representation of the phantom disposed on the patient support surface in the first rotated position; and determining, by the control device, a second difference between the second representation of the phantom and a second reference contour of the phantom.

6. A method according to item 5, further comprising:

rotating the phantom about a second axis such that the phantom is disposed on the patient support surface in a second rotated position; obtaining, by the control device, a third through-plane distorted MR image comprising a third representation of the phantom disposed on the patient support surface in the second rotated position; determining, by the control device, a third difference between the third representation of the phantom and a third reference contour of the phantom.

7. A method according to any preceding item, comprising:

obtaining, by the control device, respective through-plane distorted MR images in each of three perpendicular planes, the respective through-plane distorted MR images comprising respective representations of the phantom disposed on the patient support surface; and determining, by the control device, respective differences between the respective representations of the phantom and respective reference contours of the phantom.

8. A method according to any preceding item, wherein the phantom is disposed offset from the isocentre of the radiotherapy device.

9. A method according to any preceding item, comprising fixing the phantom on the patient support surface using a fixation mechanism.

10. A method according to any preceding item, wherein the phantom comprises an outer surface tapered along at least one axis of the phantom between a base and a tapered end.

11. A method according to item 10 when dependent on item 9, comprising fixing, using the fixation mechanism, the phantom on the patient support such that the tapered end of the phantom is selectively aligned at different respective times with each of three perpendicular axes of the radiotherapy device.

12. A method according to any preceding item, wherein the outer surface of the phantom has a pyramidal shape, and optionally wherein the pyramidal shape has a square base.

13. A method according to any preceding item, comprising determining a location of the phantom by imaging MV markers comprised in the phantom.

14. A computer-readable medium comprising computer-executable instructions which, when executed by a processor, cause performance of the method of any preceding item.

15. A phantom for use in evaluating contour display accuracy for radiotherapy, the phantom comprising:

an outer surface tapered along at least one axis of the phantom between a base and a tapered end; and a fixation mechanism configured to fix the phantom on a patient support surface of a radiotherapy device such that the tapered end is selectively alignable at different respective times with each of three perpendicular axes of the radiotherapy device.

16. A phantom according to item 15, wherein the outer surface of the phantom comprises a pyramidal shape, and optionally wherein the pyramidal shape has a square base.

17. A phantom according to item 15 or item 16, wherein the tapered end is a first tapered end, and wherein the outer surface comprises a second tapered end oriented perpendicular to the first tapered end, and a third tapered end oriented perpendicular to the first tapered end and the second tapered end.

18. A phantom according to any of items 15-17, wherein the fixation mechanism is configured to couple to a second fixation mechanism on the patient support surface, and optionally wherein the second fixation mechanism comprises an index bar.

19. A phantom according to item 18, wherein the phantom comprises a rotation holder for coupling to the second fixation mechanism on the patient support surface via a support bar.

20. A phantom according to any of items 15-19, wherein the phantom has a hollow cavity configured to receive and retain liquid.

21. A phantom according to any of items 15-20, wherein the phantom comprises MV markers.

22. A phantom according to any of items 15-21, wherein the phantom is made of MR-compatible plastic.

23. A phantom according to any of items 15-22, wherein the phantom comprises a dosimetric film insert cavity for receiving a dosimetric film.

24. A phantom according to any of items 15-23, wherein the phantom comprises one or more handles on the outer surface thereof for enabling lifting of the phantom by a user.

25. A phantom according to any of items 15-24, wherein the three perpendicular axes comprise a first axis oriented into-out of a bore of the radiotherapy device, a second axis oriented from a left side to a right side of the radiotherapy device and a third axis oriented from a bottom to a top of the radiotherapy device.

**Claims**

1. A method for evaluating contour display accuracy for radiotherapy, the method comprising:

   obtaining, by a control device, a through-plane distorted MR image comprising a representation of a phantom disposed on a patient support surface of a radiotherapy device; and
   determining, by the control device, a difference between the representation of the phantom and a reference contour of the phantom.

2. A method according to claim 1, wherein the reference contour of the phantom comprises a ground truth contour of the phantom which is not distorted.

3. A method according to claim 1, wherein the reference contour of the phantom comprises a distortion-corrected contour of the phantom.

4. A method according to claim 1, wherein the reference contour of the phantom comprises a distortion-corrected contour of the phantom which has been warped such that it exhibits through-plane distortion.

5. A method according to any preceding claim, further comprising:

   rotating the phantom about a first axis such that the phantom is disposed on the patient support surface in a first rotated position;
   obtaining, by the control device, a second through-plane distorted MR image comprising a second representation of the phantom disposed on the patient support surface in the first rotated position; and
   determining, by the control device, a second difference between the second representation of the phantom and a second reference contour of the phantom.

6. A method according to claim 5, further comprising:

   rotating the phantom about a second axis such that the phantom is disposed on the patient support surface in a second rotated position;
   obtaining, by the control device, a third through-plane distorted MR image comprising a third representation of the phantom disposed on the patient support surface in the second rotated position;
   determining, by the control device, a third difference between the third representation of the phantom and a third reference contour of the phantom.

7. A method according to any preceding claim, comprising:

   obtaining, by the control device, respective through-plane distorted MR images in each of three perpendicular planes, the respective through-plane distorted MR images comprising respective representations of the phantom disposed on the patient support surface; and
   determining, by the control device, respective differences between the respective representations of the phantom and respective reference contours of the phantom.

8. A method according to any preceding claim, wherein the phantom is disposed offset from the isocentre of the radiotherapy device.

9. A method according to any preceding claim, comprising fixing the phantom on the patient support surface using a fixation mechanism.

10. A method according to any preceding claim, wherein the phantom comprises an outer surface tapered along at least one axis of the phantom between a base and a tapered end.

11. A method according to claim 10 when dependent on claim 9, comprising fixing, using the fixation mechanism, the phantom on the patient support such that the tapered end of the phantom is selectively aligned at different respective times with each of three perpendicular axes of the radiotherapy device.

12. A method according to any preceding claim, wherein the outer surface of the phantom has a pyramidal shape, and optionally wherein the pyramidal shape has a square base.

13. A method according to any preceding claim, comprising determining a location of the phantom by imaging MV markers comprised in the phantom.

14. A computer-readable medium comprising computer-executable instructions which, when executed by a processor, cause performance of the method of any preceding claim.

**15.** A phantom for use in evaluating contour display accuracy for radiotherapy, the phantom comprising:

an outer surface tapered along at least one axis of the phantom between a base and a tapered end; and
a fixation mechanism configured to fix the phantom on a patient support surface of a radiotherapy device such that the tapered end is selectively alignable at different respective times with each of three perpendicular axes of the radiotherapy device.

Fig. 1

Fig. 2

Fig. 3

Fig. 4b

Fig. 4a

Fig. 5

Fig. 6

Fig. 7

Fig. 8

900

**902**
Request an MR stack-offset position which represents the target position in the patient co-ordinate system

**904**
Apply an MR-MV transform to determine the target position in MV space

**906**
Localise the target position in the phantom space based on its position in MV space and phantom geometry

**908**
Obtain a through-plane distorted MR image of the phantom on the patient support surface

**910**
Determine a reference contour of the phantom

**912**
Compare the through-plane distorted MR image of the phantom and the reference contour of the phantom

# Fig. 9

Fig. 10a

Fig. 10b

1100

**1150**
Treatment device

Computing system
**1110**

Controller circuitry

1111

Training circuitry

1118

Memory

1113

Image processing circuitry

1119

Network interface circuitry

1115

Input/output circuitry

1117

1160

1170

1180

Image acquisition device
**1140**

Input device
**1120**

Output device
**1130**

# Fig. 11

1200

1210

Fig. 12